# EUROPEAN PATENT SPECIFICATION

(11) **EP 2 088 152 B1**
(45) Date of publication and mention of the grant of the patent: **01.02.2012**
(21) Application number: 07852031.9
(22) Date of filing: 24.10.2007
(51) Int. Cl.: C07K 5/062, C07J 63/00, C07J 53/00, A61K 38/05, A61P 31/18

(54) **N'-{N-[3-OXO-LUPEN-28-OYL]-9-AMINONONANOYL}-3-AMINO-3-PHENYLPROPEONIC ACID AND THE PHARMACEUTICALLY ACCEPTABLE DERIVATIVES THEREOF, A METHOD FOR THE PRODUCTION AND THE USE THEREOF IN THE FORM OF A MEDICINAL AGENT**
N'-{N-[3-OXO-LUPEN-28-OYL]-9-AMINONONANOYL}-3-AMINO-3-PHENYLPROPEONSÄURE UND PHARMAZEUTISCH AKZEPTABLE DERIVATE DAVON, VERFAHREN ZU IHRER HERSTELLUNG UND IHRE VERWENDUNG ALS MEDIZINISCHE WIRKSTOFFE
ACIDE N'-{N-[3-OXO-LUPAN-28-OIL]-9-AMINONONANOIL}-3-AMINO-3-PHÉNYLPROPIONIQUE ET SES SELS PHARMACEUTIQUEMENT ACCEPTABLES, PROCÉDÉ DE FABRICATION ET SON UTILISATION EN TANT QUE MÉDICAMENT

(30) Priority: 30.10.2006 RU 2006138356
(43) Date of publication of application: 12.08.2009
(73) Proprietor: Institut Neftekhimii I Kataliza Rossiiskoi Akademii Nauk, Ufa 450075 (RU)
(72) Inventor: DZHEMILEV, Usein Memetovich, Ufa, 450054 (RU); TOLSTIKOV, Genrikh Alexandrovich, Novosibirsk, 630090 (RU); POKROVSKY, Andrei Georgievich, 630559 P. Koltsovo (RU); SALAKHUTDINOV, Nariman Faridovich, Novosibirsk, 630090 (RU); TOLSTIKOVA, Tatyana Genrikhovna, Novosibirsk, 630090 (RU); SHULTS, Elvira Eduardovna, Novosibirsk, 630117 (RU)
(74) Representative: Hart-Davis, Jason
(86) International application number: PCT/RU2007/000587
(87) International publication number: WO 2008/054250

(56) References cited:
- RU-C1- 2 211 843
- US-A- 5 468 888
- US-B1- 6 369 109
- TOLSTIKOVA T G ET AL: "Biological activity and pharmacological prospects of lupane terpenoids: II. Semisynthetic lupane derivatives" RUSSIAN JOURNAL OF BIOORGANIC CHEMISTRY, KLUWER ACADEMIC PUBLISHERS-PLENUM PUBLISHERS, NE, vol. 32, no. 3, 26 May 2006 (2006-05-26), pages 261-276, XP019406864 ISSN: 1573-9163

## Description

### Technical Field

The invention relates to organic chemistry and medicine, in particular to virology. More specifically the invention relates to a novel chemical compound N'-{N-[3-oxo-lupan-28-oyl]-9-aminononanoyl}-3-amino-3-phenylpropionic acid of formula **1** and to the pharmaceutically acceptable salts and prodrug forms thereof exhibiting an anti-viral (anti-HIV) and immunostimulating activity:

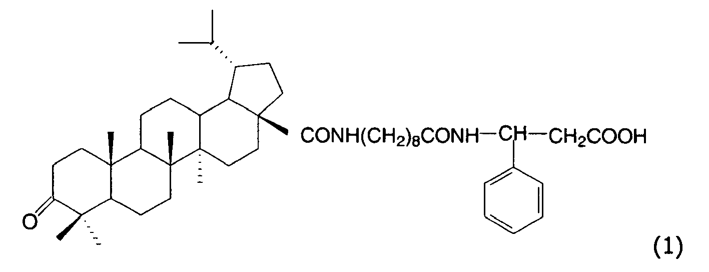

The combination of anti-viral and immunostimulating activities makes these compounds promising for the use in medicine as an anti-viral and immunostimulating medicinal agent.

### Background Art

The derivatives of the lupane triterpenes are currently being actively studied as anti-viral (anti-HIV) agents. The synthesis of the whole series of derivatives of betulinic acid (3β-hydroxy-lupen-20(29)-28-oic) acid is described. In particular, 3-O-acylates are synthesized, among which dimethyl succinates 2, 3, dipeptides, **4**, for example, as well as bifunctional derivatives, compound **5**, for example, stand out. [Cichewicz R.H., Kouzi S.A., Med.Res.Rev.,2004,V.24,N1,90; Huang L., Ho Ph., Lee K.-H., Chen Ch.-H, Bioorg.Med.Chem.,2006,14,2279].

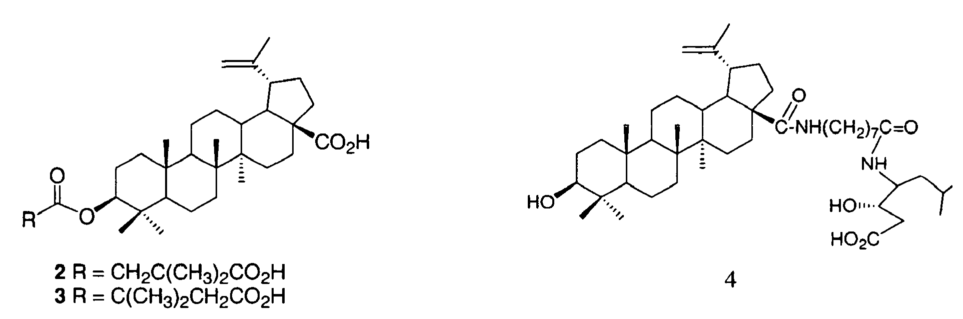

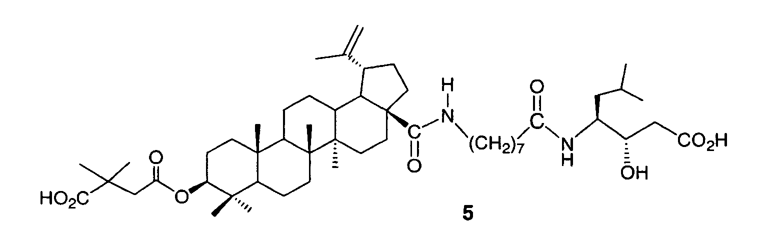

An approach which can expand the range of agents which are promising for developing anti-HIV products is the use of betulonic (3-oxo-lupen-20(29)-28-oic) acid **6** as a parent compound. This substance is synthesized easier than betulinic (3β-hydroxy-lupen-20(29)-28-oic) acid **7,** inasmuch as it is an intermediate product in the synthesis scheme of betulinic acid from betulin **8.** The latter is obtained by extracting birch bark with a yield of up to 25% [Krasutsky P.A., Nat.Prod.Rep., 2006]

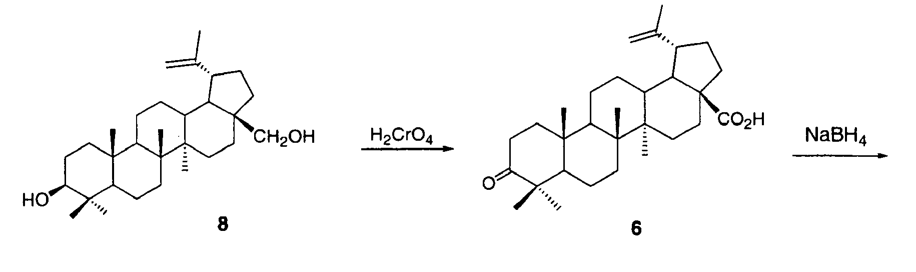

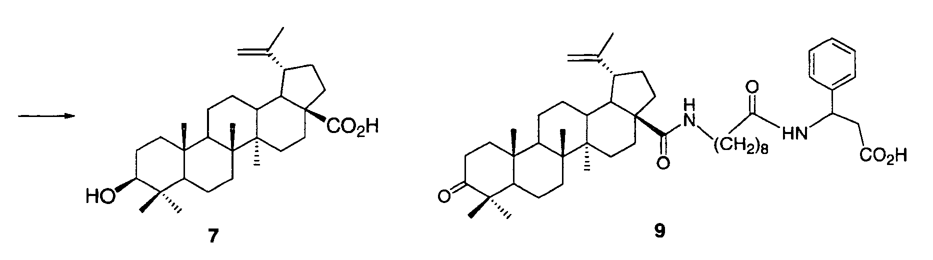

The anti-viral activity of betulonic acid and the derivatives thereof is poorly studied. It is known that dipeptide of betulonic acid having a structure of N'-{N-[3-oxo-lupen-20(29)-28-oyl-9-aminononanoyl}-3-amino-3-phenylpropionic acid **9** exhibits immunostimulating and anti-viral activity [Patent RU 2211843, Bulletin "Inventions. Utility Models" No. 25, 2003].

This compound described earlier by the inventors can be considered as the closest analog of the compound of the present invention.

### Disclosure of Invention

The objective of the present invention is enlarging the arsenal of highly active anti-viral agents and the preparation of compounds exhibiting a higher anti-HIV activity as compared to the analogs. The assigned objective is attained by novel chemical compounds prepared on the basis of dihydrobetulonic acid (3-oxo-lupan-28-oic) acid **10,** in particular, N'-{N-[3-oxo-lupan-28-oyl-9-aminononanoyl}-3-amino-3-phenylpropionic acid **1** and salts and prodrug forms thereof exhibiting high anti-viral and immunostimulatory effect.

Thus, in its first aspect the present invention relates to N'-{N-[3-oxo-lupan-28-oyl-9-aminononanoyl}-3-amino-3-phenylpropionic acid 1 of formula **1**: 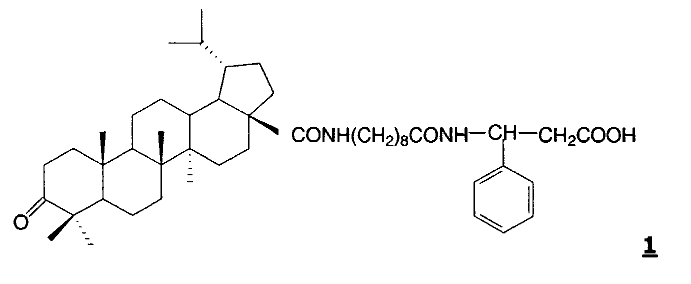 or a pharmaceutically acceptable salt or a prodrug form thereof. In one of the embodiments, a pharmaceutically acceptable salt is a lithium, ammonium, sodium, potassium or calcium salt.

In the next aspect the present invention relates to a method for the production of N'-{N-[3-oxo-lupan-28-oyl-9-aminononanoyl}-3-amino-3-phenylpropionic acid or a pharmaceutically acceptable salt or a prodrug form thereof. The method according to the present invention comprises the following steps:
a) providing starting materials containing betulin **8**;
b) subjecting betulin of formula **8** to catalytic hydrogenation to form dihydrobetulin **15;**
c) oxidizing dihydrobetulin **15** to form dihydrobetulonic acid **10;**
d) isolating and purifying the formed dihydrobetulonic acid **10** through a salt;
e) treating the obtained purified dihydrobetulonic acid **10** with oxalyl chloride to form acyl chloride **11;**
f) condensing acyl chloride **11** with 9-aminononanoic acid to produce amide **12;**
g) bringing the resulting amide **12** in a reaction with 3-phenyl-3-aminopropionic acid methyl ester to form dipeptide methyl ester **13;**
h) hydrolyzing dipeptide methyl ester **13** to produce the acid of formula **1;**
i) optionally converting the acid of formula **1** into a pharmaceutically acceptable salt or a prodrug form.

In one of the embodiments of the method a betulin compound or a total product of birch bark extraction containing 85-90% of betulin and 8-10% of lupeol **14** is used as a starting material. In the next embodiment of the method, the oxidation of dihydrobetulin is carried out with chromic acid. In another embodiment, the production of a pharmaceutically acceptable salt is carried out by reacting the acid **1** with an equivalent amount of an alkaline metal hydroxide or an alkaline-earth metal hydroxide or ammonium hydroxide in a solvent chosen from among lower alkyl alcohols or a mixture thereof.

In its another aspect, the present invention relates to a pharmaceutical composition comprising an effective amount of N'-{N-[3-oxo-lupan-28-oyl-9-aminononanoyl}-3-amino-3-phenylpropionic acid or a pharmaceutically acceptable salt or a prodrug form thereof together with a pharmaceutically acceptable carrier, diluent or excipient.

At last in one more aspect the present invention concerns the use of N'-{N-[3-oxo-lupan-28-oyl-9-aminononanoyl}-3-amino-3-phenylpropionic acid or a pharmaceutically acceptable salt or a prodrug form thereof for the manufacture of a medicinal agent exhibiting an anti-viral (anti-HIV) and immunostimulating activity.

An undoubted surprising advantage of the compound of the present invention, salts and prodrug forms thereof is a possibility to use as a peroral anti-viral (anti-HIV) medicinal agent which results from the low values of inhibiting concentrations towards the virus.

The novel compound - dipeptide **1** having a structure of N'-{N-[3-oxo-lupan-28-oyl-9-aminononanoyl}-3-amino-3-phenylpropionic acid is non-toxic, exhibits an anti-viral (anti-HIV) activity and an immunostimulating effect.

The method of the present invention provides obtaining a novel compound - dipeptide **1** with a high yield, wherein the locally available raw material - betulin produced by extracting birch bark (elm) that is a large-capacity waste product of wood industry and that does not find wide qualified application at the present time can be used as a starting material for synthesis.

### Modes for Carrying out the Invention

### Definitions

The term "pharmaceutically acceptable salts" includes derivatives of the compound of the present invention, wherein the parent compound is modified by preparing nontoxic salts thereof with bases, as well as relates to pharmaceutically acceptable solvates of the compound of the present invention and the salts thereof.

The appropriate pharmaceutically acceptable salts comprise commonly used nontoxic salts, for example, salts of inorganic bases, for example, salts of alkaline metals (for example, lithium, sodium, potassium salts), salts of alkaline-earth metals (for example, calcium, magnesium salts), ammonium salts; or salts of organic bases, for example, amine salts (e.g. salts of methylamine, dimethylamine, cyclohexylamine, benzylamine, piperidine, ethylendiamine, ethanolamine, diethanolamine, triethanolamine, tris(hydroxymethylamino)ethane, monomethylmonoethanolamine, procaine and caffeine), salts of basic amino acids (e.g. arginine salts and lysine salts), tetraalkylammonium salts and others which can be formed by reacting with the carboxyl group of the compound of the present invention. The list of some other appropriate salts can be found in Remington's Pharmaceutical Sciences, 17th ed., Mack Publishing Company, Easton, Pa, p. 1418 (1985).

The pharmaceutically acceptable salts according to the present invention can be synthesized by the standard chemical methods known to one skilled in this art, for example, from a free acidic form of the compound of the present invention and a base or by the interconversion of salts. As a rule, such salts can be obtained by reacting a free acid form with a stoichiometric amount of the relevant base. Such reactions are typically carried out in water or in an organic solvent or a mixture thereof. As a rule, non-aqueous media like ether, ethyl acetate, ethanol, isopropanol or acetonitrile are preferred.

The term "a prodrug" or "a prodrug form" of the compound of the present invention comprises any compounds which become the compounds of formula 1 upon administering to a mammal, for example, after metabolic conversion of a prodrug. In the context of the present invention, the term "a prodrug" comprises such functional derivatives of the compound of the present invention as esters and amides.

The examples of esters comprise aliphatic esters, for example, esters of lower alkyls, e.g. methyl ester, ethyl ester, propyl ester, isopropyl ester, butyl ester, isobutyl ester, tert-butyl ester, pentyl ester and 1-cyclopropylethyl ester; lower alkenyl esters, e.g. vinyl ester and allyl ester; lower alkynyl esters, e.g. ethinyl ester and propynyl ester; esters of lower hydroxyalkyls, e.g. hydroxyethyl ester; esters of lower alkoxyalkyls, e.g. metoxymethyl ester and 1-metoxyethyl ester; esters of optionally substituted aryls, for example, phenyl ester, tolyl ester, tert-butylphenyl ester, salicylic ester, 3,4-dimetoxyphenyl ester and benzamidophenyl ester; and esters of lower arylalkyls, e.g. benzyl ester, trityl ester and benzhydryl ester.

"Amide" means a group represented by the formula - CONR'R", wherein each of R' and R" denotes a hydrogen atom, lower alkyl, aryl, alkyl- or arylsulfonyl, lower alkenyl or lower alkynyl and covers, for example, lower alkylamides, such as methylamide, ethylamide, dimethylamide and diethylamide; arylamides, such as anylide or toluidine; and such alkyl- or arylsulfonyls as methylsulfonylamide, ethylsulfonylamide and tolylsulfonylamide.

The term "a therapeutically effective amount" of the compound of the present invention means an effective amount when it is administered to a human being or a non-human patient to gain a favorable therapeutic effect such as relief of symptoms, for example, an amount effective to relieve the symptoms of a viral infection and preferably an amount sufficient for the relief of HIV infection symptoms. In some cases, in a patient suffering from HIV infection the infection symptoms can be absent. Thus, a therapeutically effective amount of the compound is also an amount sufficient to prevent significant increase in or to substantially reduce the detectable level of virus and antibodies to the virus in blood, serum or tissues of a patient. Significant increase or reduction in the detectable level of virus or antibodies to the virus is any detectable change which is statistically significant when using the standard parametric criterion such as, for example, Student's t-test, wherein p<0.05.

The synthesis of the compound of formula I can be carried out in accordance with the following Scheme 1: betulin **8** (or a total product of extracting birch bark containing 85-90% of betulin and 8-10% of lupeol **15**) is subjected to catalytic hydrogenation resulting in dihydrobetulin **16** (or a mixture thereof with dihydrolupeol **17**). Oxidation, for example, with chromic acid then ensues to yield dihydrobetulonic acid **10**, or a mixture thereof with lupanone **18**. Isolation and purification of acid **10** is carried out through a salt. The second useful product (if a natural mixture of triterpenes is used) is lupanone **18**.

The treatment of dihydrobetulonic acid **10** with oxalyl chloride yields acyl chloride **13**, by the condensation of which with 9-aminononanoic acid amide **12** was prepared. The reaction of obtained amide **12** with 3-phenyl-3-aminopropionic acid methyl ester results in producing dipeptide methyl ester **14**, by hydrolysis of which the desired product I in the form of a free acid is obtained.

Optionally, the free acid of formula I can be converted into a pharmaceutically acceptable salt (for example, a lithium, ammonium, sodium, potassium, magnesium or calcium salt) by the method known to persons skilled in this field of art, for example, by neutralizing dipeptide I with appropriate hydroxides.

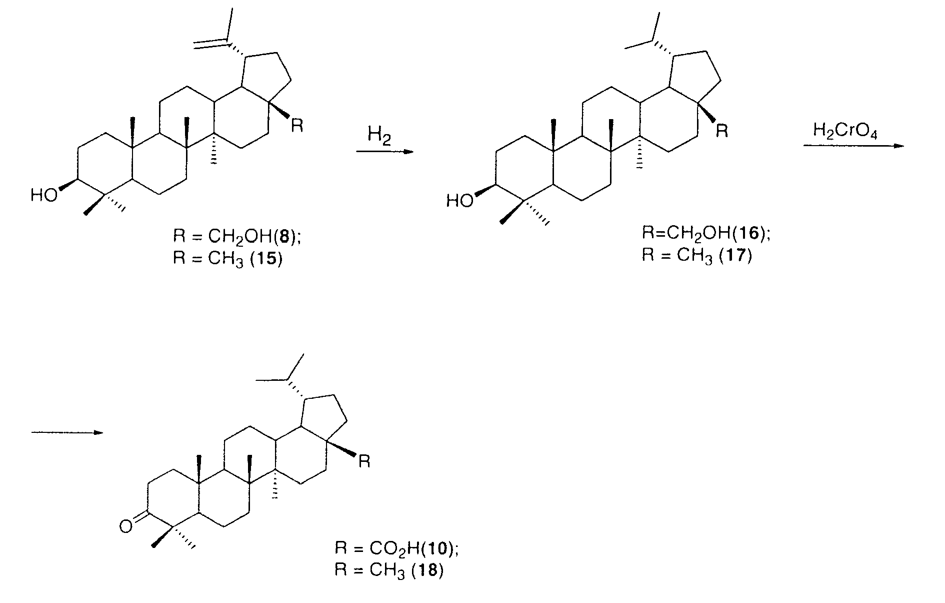

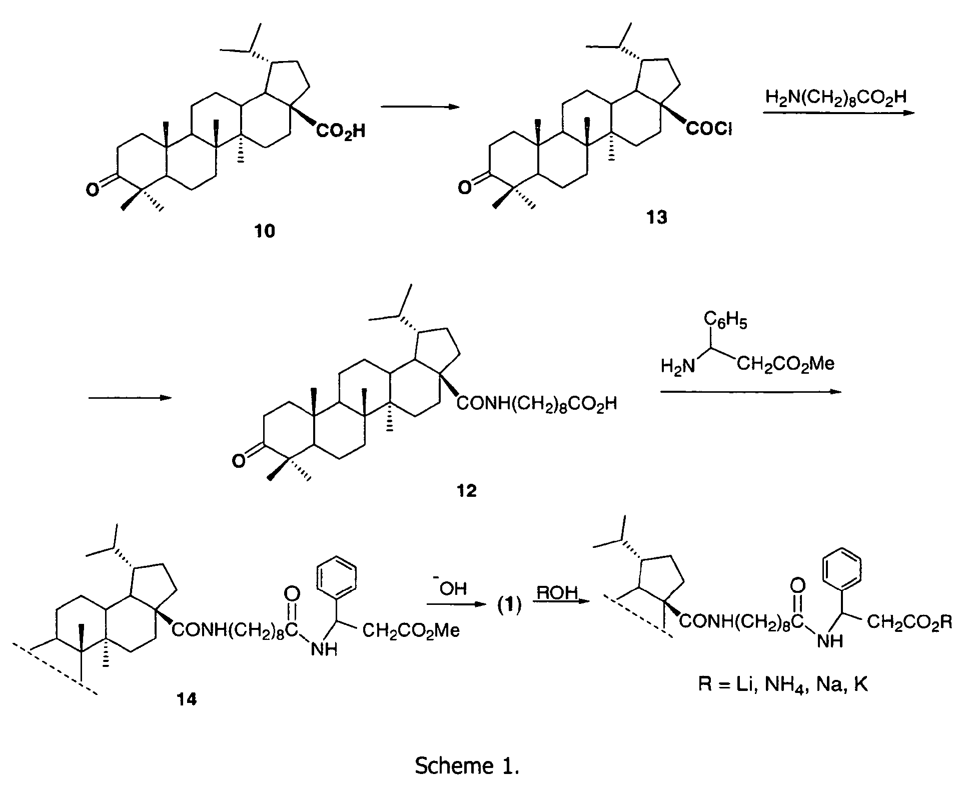

### Pharmaceutical preparations

The compound of formula 1, a salt or a prodrug form thereof can be administered in the form of a pure chemical substance, but preferably, they are administered in the form of a pharmaceutical composition or a preparation. Correspondingly, the invention relates to pharmaceutical compositions containing the compound of formula 1 and/or a pharmaceutically acceptable salt and/or a prodrug form thereof together with one or more pharmaceutically acceptable carriers, adjuvants, diluents or other ingredients.

The compound of the present invention can be administered perorally, topically, parenterally, with inhalation or spray, sublingually, transdermally, rectally, in the form of an eye ointment or by other methods, in the standard dosage forms containing conventional nontoxic pharmaceutically acceptable carriers, adjuvants and media.

Besides the desired compound, the compositions of the invention can contain a pharmaceutically acceptable carrier, one or more compatible solid or liquid fillers, diluents or encapsulating agents appropriate for the administration to a human or other animal. The carriers should be sufficiently pure and sufficiently low-toxic so that to regard them appropriate for the administration to an animal being treated. A carrier can be inert or can have its own pharmaceutically favorable properties. An amount of the carrier used in combination with the compound is sufficient to provide practical quality of the material to be administered per unit dose of the compound.

The examples of pharmaceutically acceptable carriers and components thereof are sugars, such as lactose, glucose and saccharose; starches, such as corn starch and potato starch; cellulose and derivatives thereof, such as sodium carboxymethylcellulose, ethylcellulose and methylcellulose; powdered tragacanth gum; gelatine; talc; solid lubricants such as stearinic acid and magnesium stearate; calcium sulfate, vegetable oils, such as peanut butter, cottonseed oil and corn oil; polyols, such as propylene glycol, glycerin, sorbitol, mannitol and polyethylene glycol; alginic acid; emulsifiers, such as tweens; wetting agents, such as sodium laurylsulfate; dyes; correctives; pelletizing agents; stabilizers; antioxidants; preservatives; pyrogen-free water; isotonic physiological solution, glucose solution and phosphate-buffered solutions.

In particular, pharmaceutically acceptable carriers for systemic administration comprise sugars, starches, cellulose and derivative thereof, malt, gelatin, talc, calcium sulfate, vegetable oils, synthetic oils, polyols, alginic acid, phosphate-buffered solutions, emulsifiers, isotonic physiological solution and pyrogen-free water. The preferred carriers for parenteral administration are propylene glycol, ethyl oleate, pyrrolidone, ethanol and sesame oil.

Optional active agents which do not significantly affect the activity of the compound of the present invention can be added to the pharmaceutical composition.

One or more compounds of the invention including pharmaceutically acceptable salts, esters or amides, are mixed at effective concentrations with an appropriate pharmaceutical carrier, excipients, an adjuvant or a medium. In the cases when the compounds exhibit insufficient solubility solubilization methods can be used. Such methods are known to ones skilled in this field of art and comprise the use of co-solvents, such as dimethylsulfoxide (DMSO), the use of surfactants, such as tween, or dissolving in an aqueous solution of sodium bicarbonate and other methods.

After admixing or adding the compound(s) of the present invention the resulting mixture can represent a solution, suspension, emulsion, or a similar preparation. The form of the resulting mixture depends on some factors, including the prospective route of administration and the compound solubility in the chosen carrier or medium. The effective concentration sufficient to relieve the symptoms of a disease, disorder or condition which is being treated can be determined empirically.

The pharmaceutical compositions containing the compounds of the general formula 1 can be in the form suitable for peroral administration, for example, in the forms of tablets or granules, pastilles, troches, suspensions in water or oil, dispersible powders or granules, emulsions, hard or soft capsules, or syrups or elixirs. The compositions intended for peroral administration can be prepared according to any method known in the art for the preparation of pharmaceutical compositions, and these compositions can contain one or more agents such as sweeteners, correctives, flavoring agents, dyes and preservatives so as to obtain products attractive in appearance and pleasant for ingestion.

Peroral preparations contain 0.1 to 99% of the inventive compound and, as a rule, at least about 20% (% by weight) of the compound of the present invention. Some embodiments contain about 25% to about 50% or 5% to 75% of the inventive compound.

### Liquid preparations

The inventive compounds can be formulated into liquid preparations for peroral administration, such as suspensions in water or oil, solutions, emulsions, syrups or elixirs, for example. Besides, the preparations containing these compounds can be represented as a dry product to prepare a mixture with water or other appropriate medium before use. These liquid preparations can contain conventional additives, such as suspending agents (for example, a sorbitol syrup, methylcellulose, glucose/sugar, syrup, gelatin, hydroxyethylcellulose, carboxymethylcellulose, aluminum stearate gel and hydrogenated edible fats), emulsifiers (for example, lecithin, sorbitan monostearate or Arabic gum), non-aqueous media which can comprise edible oils (for example, almond oil, fractionated coconut oil, silyl ethers, propylene glycol and ethyl alcohol), and preservatives (for example, methyl- or n-propyl-p-hydroxy benzoate and sorbic acid).

The compositions administered perorally also comprise liquid solutions, emulsions, suspensions, powders, granules, elixirs, tinctures, syrups and the like forms. The pharmaceutically acceptable carriers suitable for the preparation of such compositions are well-known in the art. Peroral compositions can comprise preservatives, correctives, sweeteners, such as sucrose or saccharine, flavor hiding agents, and dyes.

The typical components of carriers for syrups, elixirs, emulsions and suspensions are ethanol, glycerin, propylene glycol, polyethylene glycol, sugar solution, sorbitol and water. Syrups and elixirs can comprise sweeteners, for example, glycerin, propylene glycol, sorbitol or saccharose. Such compositions can also contain a demulcent.

### Suspensions

In the case of a suspension, typical suspending agents are methylcellulose, carboxymethylcellulose sodium, avicel RC-591, tragacanth and sodium alginate; typical wetting agents are lecithin and polysorbate 80; and typical preservatives are methyl paraben and sodium benzoate.

Aqueous suspensions contain the active agent(s) in a mixture with excipients suitable for obtaining aqueous suspensions. These excipients are suspending agents, for example, carboxymethylcellulose sodium, methylcellulose, hydropropylmethyl cellulose, sodium alginate, polyvinyl pyrrolidone, tragacanth gum and Arabic gum; dispersing or wetting agents; naturally-occurring phosphatides, for example, lecithin, or products of condensation of alkylenoxide with fatty acids, for example, polyoxyethylene stearate, or products of condensation of ethylene oxide with long-chain aliphatic alcohols, for example, with heptadecaethylene oxycetanol, or products of condensation of ethylene oxide with partial esters produced from fatty acids and hexitol, such as substituted polyoxyethylene sorbitol, or products of condensation of ethylene oxide with partial esters produced from fatty acids and hexitol anhydrides, for example, substituted polyoxyethylene sorbitan. Aqueous suspensions can also contain some preservatives, for example ethyl- or n-propyl-p-hydroxybenzoate.

Oil suspensions can be prepared by suspending active ingredients in a vegetable oil, for example, peanut butter, olive oil, sesame oil and coconut oil, or in a mineral oil, such as liquid paraffin. Oil suspension can contain a thickening agent, for example, bee wax, solid paraffin or cetyl alcohol. Some sweeteners, such as mentioned above, and correctives can be added to obtain pleasant peroral preparations. These compositions can be preserved by adding an antioxidant, such as ascorbic acid.

### Emulsions

The pharmaceutical compositions of the invention can also be in the form of oil-in-water emulsions. The oil phase can represent a vegetable oil, for example, olive oil or peanut butter, or a mineral oil, for example, liquid paraffin or mixtures thereof. The appropriate emulsifiers can be naturally occurring gums, for example, Arabic gum or tragacanth gum, naturally occurring phosphatides, for example, soybean lecithin, and esters or partial esters produced from fatty acids and hexitol anhydrides, for example, sorbitan monooleate, and products of condensation of said partial esters with ethylene oxide, for example, polyoxyethylene sorbitan monooleate.

### Dispersing powders

Dispersing powders and granules suitable for preparing an aqueous suspension by adding water are the active ingredient in a mixture with a dispersing or wetting agent, suspending agent and one or more preservatives. The appropriate dispersing or wetting agents and suspending agents are the agents already exemplified above.

### Tablets and capsules

Tablets typically contain conventional pharmaceutically compatible auxiliary agents such as inert diluents, such as calcium carbonate, sodium carbonate, mannitol, lactose and cellulose; binders, such as starch, gelatin and saccharose; dispersing agents, such as starch, alginic acid and croscarmellose; lubricants such as magnesium stearate, stearic acid and talc. Glidants, such as silicon dioxide can be used to improve fluidity characteristics of a powder composition. For appearance, dyes such as FD&C can be added. Sweeteners and correctives such as aspartame, saccharine, menthol, peppermint and fruit flavors are useful as adjuvants for chewable tablets. Capsules (including sustained release and delayed release preparations) typically contain one or more solid diluents described above. The selection of carrier components often depends on secondary factors such as flavor, price and storage stability.

These compositions can also be coated using the conventional methods, typically with a pH-dependent coating, so that the desired compound is released in the gastro-intestinal tract in the proximity to the desired administration site or at different time points to sustain the desired effect. Such dosage forms typically comprise one or more components from among cellulose acetate phthalate, polyvinyl acetate phthalate, hydroxypropylmethylcellulose phthalate, ethyl cellulose, Eudragit coatings, waxes and shellac and other materials.

Preparations for peroral administration can be formulated into hard gelatin capsules, wherein the active ingredient is mixed with an inert solid diluent, for example, calcium carbonate, calcium phosphate and kaolin, or in the form of soft gelatin capsules, wherein the active ingredient is mixed with water or an oil medium, for example, peanut butter, liquid paraffin or olive oil.

### Injectable and parenteral compositions

Pharmaceutical compositions can be in the form of a sterile water or oil suspension for injections. Such suspension can be prepared according to the prior art using appropriate dispersing or wetting agents and suspending agents mentioned above. A sterile preparation for injections can also be a sterile solution for injections or a suspension for injections in a non-toxic parenterally acceptable diluent or solvent, for example, in the form of a solution in 1,3-butandiol. Among acceptable media and solvents, which can be used, are water, Ringer solution and an isotonic sodium chloride solution . Moreover, sterile nonvolatile oils are usually used as a solvent or a suspending medium. For this purpose any mixture of nonvolatile oils can be used including mono- and diglycerides. Moreover, for obtaining preparations for injections fatty acids such as oleinic acid are applicable.

The compounds of formula 1 can be administered parenterally in a sterile medium. The parenteral administration comprises hypodermic injections, intravenous, intramuscular and intrathecal injections or infusion methods. A medicinal agent, depending on the medium and concentrations used, can be either suspended or dissolved in the medium. Dissolving in a medium of adjuvants, such as local anesthetics, preservatives and buffering agents can be advantageous. In the compositions for parenteral administration a carrier amounts to at least around 90% by weight of the total composition.

### Suppositories

The compounds of the present invention can also be administered in the form of suppositories for rectal or vaginal administration of a medicinal agent. Such compositions can be prepared by mixing the medicinal agent with an appropriate non-irritating excipient which is solid at ordinary temperatures, but liquid at rectal temperature and will, consequently, melt in the rectum releasing a medicinal agent. These agents are cocoa oil and polyethylene glycols.

### Preparations for topical application

The compounds of the inventions can be formulated into a composition for local or topical application, for example, for topical application on skin or mucous membranes such as eyes, in the form of gels, creams and lotions for application to eyes or for intracisternal or intraspinal application. Topical compositions of the present inventions can be in any form including, for example, in the form of solutions, creams, ointments, gels, lotions, milk, cleansing refreshers, wetting agents, sprays, dermal patches and the likes.

Such solutions can be prepared with appropriate salts in the form of 0.01%-10% isotonic solutions, pH is about 5-7. The inventive compounds can also be formulated into a composition for transdermal administration in the form of transdermal patches.

The compositions for topical application comprising the active compound can be mixed with different carrier materials well known in the art such as, for example, water, alcohols, aloe vera gel, allantoin, glycerin, oils with vitamins A and E, a mineral oil, propylene glycol PPG-2, myristyl propionate and the likes.

Other materials for application in the carriers for topical compositions are, for example, emollients, solvents, wetting agents, thickeners and powders. Examples of each of said material types which can be used separately or mixed with one or more materials are the following agents: emollients - stearyl alcohol, glycerol monoricinoleate, glyceryl monostearate, propan-1,2-diol, butan-1,3-diol, mink fat, cetyl alcohol, isopropyl isostearate, stearic acid, isobutyl palmitate, isocetyl stearate, oleyl alcohol, isopropyl laurate, hexyl laurate, decyl oleate, octadecane-2-ol, isocetyl alcohol, cetyl palmitate, dimethylpolysiloxane, di-n-butylsebacate, isopropyl myristate, isopropyl palmitate, isopropyl stearate, butyl stearate, polyethylene glycol, triethylene glycol, lanolin, sesame oil, coconut oil, peanut butter, castor oil, acetylated lanolin alcohols, vaseline, mineral oil, butyl myristate, isostearic acid, palmitic acid, isopropyl linoleate, lauryl lactate, myristyl lactate, decyl oleate and myristyl myristate; propellants - propane, butane, isobutane, dimethyl ether, carbon dioxide and nitrogen oxide; solvents - ethyl alcohol, methylene chloride, isopropanol, castor oil, ethylene glycol monoethyl ester, diethylene glycol monobutyl ester, diethylene glycol monoethyl ester, dimethyl sulfoxide, dimethyl formamide, tetrahydrofuran; wetting agents - glycerol, sorbitol, sodium 1-pyrrolidone-5-carboxylate, soluble collagen, dibutyl phthalate and gelatin; and powders, such as chalk, talc, Fuller's earth, kaolin, starch, gums, colloidal silicon dioxide, sodium polyacrylate, tetraalkylammonium smectites, trialkylarylammonium smectites, chemically modified magnesium aluminum silicate, organically modified montmorrilonite clay, hydrated aluminum silicate, finely dispersed silicon dioxide, carboxyvinyl polymer, carboxymethylcellulose sodium and ethylene glycol monostearate.

The compounds of the invention can also be administered topically in the form of liposomal delivery systems such as small single layer vesicles, large single layer vesicles and multi-layered vesicles. Liposomes can be produced from a range of phospholipids, such as cholesterol, stearyl amine or phosphatidylcholines.

### Other preparations

Other compositions useful to enable systemic delivery of the desired compounds comprise sublingual and nasal dosage forms. Such compositions typically contain one or more fillers such as saccharose, sorbitol and mannitol, and binders such as Arabic gum, microcrystal cellulose, carboxymethylcellulose and hydroxypropylmethylcellulose. Glidants, lubricants, sweeteners, dyes, antioxidants and correctives described above can also be incorporated.

The compositions for inhalation typically can be formulated in a solution, suspension or emulsion, which can be administered in the form of a dry powder or in the form of an aerosol using a conventional propellent (for example, dichlorodifluoromethane and trichlorofluoromethane).

### Additional components

The compositions of the present invention can also optionally contain additional active ingredients exhibiting an anti-viral and/or immunostimulating activity.

Hereinafter the invention is illustrated by means of examples which are intended for giving a better idea of the essence of the invention as claimed but should not be regarded as limiting thereof.

### EXAMPLE 1. Production of 3-oxo-lupan-28-oic (dihydrobetulonic) acid 10 from a natural mixture of triterpenes.

A mixture of triterpenes prepared by extracting European white birch (Betula pendula L.) bark comprising 85-90% of betulin and 8-10% of lupeol in an amount of 50 g was suspended in 1 l of ethanol, placed in an autoclave and after addition of skeletal nickel produced by leaching of 20 g 40% Ni-Al alloy, hydrogenated at 100°C and under pressure of 100 atm.

The mixture was discharged, heated to boiling, and the catalyst precipitate was filtered out. The solution was evaporated in vacuum to dryness. The crystal residue containing dihydrobetulin **15** and lupanol **16** was further used without additional purification.

To 10 g of suspension of triterpenes after hydrogenation in 300 ml of acetone 25 ml of Johnson reagent (135 g CrO₃, 100 ml conc. H₂SO₄, water up to 1 I) was added over 30 min. The reaction mixture was kept under stirring for 5 hours at 0°C, then isopropanol was added to produce stable green color of the solution and the solution was poured into 1.5 l of iced water to which conc. HC1 was added to pH 2. The precipitate was sucked off, washed with acidified (HCl) water, then with water, dried and dissolved under heating in toluene or methyl-tert-butyl ether (MTBE).

The solution was filtered hot and 5% NaOH solution was poured into the cooled filtrate to give pH of water layer equal to 11. After stirring for half an hour the mixture was left for 1 hour, dihydrobetulonic acid sodium salt was filtered, washed with toluene (MTBE) and dried. The organic layer was evaporated to dryness, residual lupanol was crystallized from a mixture of methanol-CHCl₃, the yield was 0.6-0.8 g, melt. temp. 208-210°C.

The sodium salt was dissolved under heating in an aqueous alcohol, the solution was filtered hot and poured in the excess of 5% HCl. The dihydrobetulonic acid precipitate formed upon standing was sucked off and dried, the yield was 6.0-6.5 g, melt. temp. 250-252°C.

### EXAMPLE 2. Synthesis of dihydrobetulonic acid (10) from betulin.

Betulin (10 g), 300 ml ethanol were loaded into an autoclave, 1 g of 10% palladium-on-coal was added and hydrogenation was carried out at 100°C and under 100 atm. hydrogen. Dihydrobetulin **15** isolated by the conventional method from the reaction medium was suspended in 400 ml of acetone and 26 ml of Johnson reagent in 100 ml of acetone was added over 30 min, then the mixture was kept under stirring at 0°C for 7 hours. Alcohol was poured into the mixture until the yellow color of the solution turned green, then the mixture was poured into 1.5 l of cold water and strongly acidified with HCl. The raw dihydrobetulonic acid precipitate was sucked off, washed with water and dried. For purification, it was dissolved under heating in toluene or MTBE, filtered hot and 5% KOH solution was poured to the cooled filtrate to form a salt (the aqueous layer should have pH 11). The salt was filtered, washed with toluene or MTBE and dried in vacuum. The potassium salt was dissolved under heating in alcohol, the insoluble residue was filtered out, and the filtrate was poured out into the excess of diluted HCl. The dihydrobetulonic acid precipitate was washed and crystallized from alcohol, melt. temp. 253-254°C.

### EXAMPLE 3. Synthesis of N'-{N-[3-oxo-lupan-28-oyl-9-aminononanoyl}-3-amino-3-phenylpropionic acid 1.

A) 2.0 ml of oxalyl chloride was poured to 5.0 g of dihydrobetulonic acid **10** in 200 ml of dry methylene chloride, the mixture was stirred at 20°C for 5-6 hours and evaporated to dryness in vacuum at t<35°C. Fifty ml of dry methylene chloride was poured to the residue and evaporated to dryness. After repeating this operation three times 4.8 g of dihydrobetulonic acid acyl chloride **11** was produced.

In the IR-spectrum there are bands at wavenumbers 1705 cm⁻¹ and 1802 cm⁻¹ typical of a 3-oxogroup and a COCl moiety.

B) A solution (1.05 mm) of aminononanoic acid hydrochloride, 1.5 ml (10 mm) of distilled trimethylchlorosilane in 150 ml of dry methylene chloride were refluxed for 4 hours under argon atmosphere, cooled to 0°C, then 2.0 g (4.5 mm) of acyl chloride **(11)** and 1.05 ml of distilled triethylamine was added and the mixture was kept under stirring for 24 hours at 20°C. 100 ml of methylene chloride was poured to the mixture, the mixture was washed with 10% HCl, then with water and 5% NaHCO₃, with water again and after drying the solution with Na₂SO₄ it was evaporated to dryness. The residue was chromatographed on silica gel and eluted with a mixture of CHCl₃-CH₃CN=95:5.

2.1 g of N-(3-oxolupan-28-oic)-9-aminononanoic acid 12 was produced, the melt. temp. was 100-102°C.

Found by MS-analysis :
mol. weight 611, C₃₉H₆₅NO₄
Calculated: 611.

In the PMR spectrum the signals of vinyl protons at 4.57 and 4.67 ppm are absent.

C) To the solution of 1.21 g (2 mm) of amide **12i** in 150 ml of dry methylene chloride, 0.29 g of 1-hydroxy benzotriazole and 5.1 g of N,N-dicyclohexylcarbodiimide was added in inert atmosphere at 0°C and the mixture was kept under stirring for half an hour at 0°C and for 5 hours at 20°C.

To the mixture cooled to 0°C 0.55 g of 3-amino-3-phenylpropionic acid methyl ester hydrochloride and 0.38 ml of triethylamine was added, followed by stirring the mixture for 24 hours at 20°C.

The precipitate of dicyclohexyl urea formed after cooling the mixture to 0°C was filtered out and washed with cold methylene chloride. The combined filtrates were washed with 10% HCl, water, 5% NaHCO₃, water and evaporated to dryness. The residue was chromatographed on Al₂O₃ with elution with chloroform. 1.3-1.4 g of dipeptide methyl ester **13** was produced, which was dissolved in 25 ml of a mixture of methanol-tetrahydrofuran 1:2 and after adding 2 ml of 4M NaON solution was kept for 24 hours at 20°C. The mixture was poured out on a mixture of ice with diluted HCl, the precipitate was filtered out, washed with water and dried over P₂O₅.

1.2 g dipeptide **1** was produced, melt. temp. 140-143°C; [α]_{D} +10,5°
Found by MS-analysis: mol. weight 758. C₄₈ H₇₄ N₂O₅.
Calculated: 758.

In the PMR spectrum the signals of vinyl protons at 4.57 and 4.67 ppm are absent.

In the IR-spectrum there is no band at 1650 cm⁻¹ typical of an isopropylidene group.

### EXAMPLE 4. Production of dipeptide 1 salts.

An equivalent amount of methanolic solution of NH₄OH, LiOH, NaOH, KOH or Ca(OH)₂ was poured to the solution of dipeptide **1** in an appropriate volume of methanol or ethanol.

After evaporation to dryness and keeping under vacuum the corresponding salts were obtained with a quantitative yield.
Ammonium salt: a colorless powder, C₄₇H₇₃N₂O₄CO₂NH₄
Lithium salt: a colorless powder, C₄₇H₇₃N₂O₄CO₂Li
Sodium salt: a creamy powder, C₄₇H₇₃ N₂O₄CO₂Na
Potassium salt: a yellowish powder, C₄₇H₇₃N₂O₄CO₂K
Calcium salt: a colorless powder, C₄₇H₇₃N₂O4CO₂Ca ½

### EXAMPLE 5. Evaluation of dipeptide 1 cytotoxicity on cells MT-4

The cytotoxicity of the compound **1** was evaluated using a cell culture of continuous human T-lymphocytes (line MT-4). A weigh of the agent (I) was dissolved in dimethyl sulfoxide (DMSO) at a concentration of 10 mg/ml and serial dilutions were prepared in a growth medium, then aliquots of the appropriate dilutions were added into wells of 96-well plates (each dilution in triplicate) to the final concentrations from 0.1 to 100 µg/ml while inoculating the cells. The inoculation concentration was 0.5x10⁶ cells/ml. The experimental (with the preparation added) and control (without the preparation added) cells were cultivated in 96-well cell culture plates manufactured by "Costar" (USA) in a growth nutrient medium RPMI-1640 containing 10% fetal calf serum from "ICN" (USA), 0.06% L-glutamine, 100 µg/ml gentamycin and 60 µg/ml lincomycin, in the presence of 5% CO₂ for 4 days at 37°C. On completion of the incubation the proportion of viable cells was counted in a Gorjaev's chamber after staining with a 0.4% trypan blue solution. A dose-dependence plot was constructed, from which a cytotoxic dose (CD₅₀) corresponding to the compound concentration reducing by 50% the proportion of viable cells compared to the control was determined. CD₅₀ for compound **1** is 6.8 µM (Table 1). Azidothymidine (AZT) and earlier described dipeptide **9** were used as reference agents.

**Table 1. Cytotoxicity of compound 1 of the present invention compared to the known anti-viral agents.**

| Compound | CD₅₀, µM |
|---|---|
| 1 | 6.8 |
| 9 | 5.6 |
| AZT | 40 |

### EXAMPLE 6. Evaluation of anti-HIV activity of dipeptide 1 when the preparation is added after the virus adsorption.

The Anti-HIV activity of dipeptide 1 was studied on a highly HIV-sensitive cell culture MT-4. The MT-4 cells were infected with a virus stock (strain HIV-1/EVK [Karamov E. V., Bogomolov B. P., Zhdanov V. M.// Review of the centers collaborating with the WHO for viral inventions, 1986, 4 p.]) at a multiplicity of infection of 0.2-0.5 tissue culture infective dose per cell and incubated at 37°C for 1 h (virus adsorption). A suspension of the infected cells was diluted with a growth nutrient medium RPMI-1640 containing 10% fetal calf serum from "ICN" (USA), 0.06% L-glutamine, 100 µg/ml of gentamycin and 60 µg/ml of lincomycin to give an inoculation concentration of 0.5x10⁶ cell/ml and added into the wells of a 96-well culture plate. Then, aliquots of serial dilutions of dipeptide **1** in DMSO were added into the wells to the final concentrations of 0.1 ng/ml to 1 µg/ml (each concentration in triplicate). The experimental (with the preparation added) and control (without the preparation added) infected cells were cultivated at 37°C and 5% CO₂ for 4 days. On completion of the incubation the proportion of viable cells was counted in a Gorjaev's chamber after staining with a 0.4% trypan blue solution and the accumulation level of virus-specific protein p24 was controlled by the immunoenzyme method as described [Fedyuk N. V., Konovalov E. E., Loktev V. B., Uryvaev L. V., Kulyandin S. A., Pokrovsky A. G.// Problems of Virology, 1992, v. 3, p. 135]. Based on the experimental quantitative data a dose-dependence plot was constructed, from which an inhibiting dose (ID₅₀) was determined, that corresponds to the concentration of dipeptide 1 reducing by 50% the accumulation of the virus antigen p24 compared to the control. The inhibiting dose for dipeptide 1 is 0.4 µM. The dose of the compound protecting the cells from death (PD₅₀) was also determined. The therapeutic selectivity index (IS) calculated as a ratio of the cytotoxic dose to the inhibiting dose (CD_{50/}ID₅₀) for dipeptide **1** amounts to 34. Additionally, the selectivity index (IS) calculated as a ratio of the toxic dose of dipeptide **1** to the protection dose thereof PD₅₀ (CD₅₀/ PD₅₀) was determined. The quantitative data of inhibiting virus reproduction with the compound **1** of the present invention and the known anti-viral compounds are listed in Table 2.

Thus, dipeptide **1** not only effectively inhibits HIV-1 but also protects cells from a virus-induced cytopathic effect (Table 2).

**Table 2. Anti-HIV activity of dipeptide 1 added after virus adsorption**

| Compound | Inhibition of P24 accumulation | | IS | Protection of cells from death |
|---|---|---|---|---|
| | ID₅₀, µM | ID₉₀, µM | | PD₅₀, µM |
| **1** | 0,2 | Not achieved | 28,0 | 0,25 |
| **9** | 0,4 | Not achieved | 14,0 | 0,5 |
| AZT | 0,014 | 0,062 | 2857,0 | No data |

### EXAMPLE 7. Evaluation of anti-HIV activity of dipeptide 1 when the preparation is added simultaneously with the virus.

The anti-HIV activity of dipeptide **1** was studied on a highly HIV-sensitive cell culture MT-4. The MT-4 cells were added into the wells of a 96-well culture plate and infected with a virus stock (strain HIV-1/EVK) at a multiplicity of infection of 0.2-0.5 tissue culture infective dose per cell, then the test substance was added into a cell suspension simultaneously with the virus to give the final concentration of 0.00001 to 1 µg/ml (each concentration in triplicate) and incubated at 37°C for 1 h (virus adsorption). On completion of the incubation, the suspension of the infected cells was diluted with a complete growth medium with all supplements as described above containing the preparations in the same concentrations to give the inoculation concentration of 0.5x10⁶ cells/ml.

The experimental (with the preparation added) and control (without the preparation added) infected cells and control uninfected cells (without the preparation added) were cultivated at 37°C and 5% CO₂ for 4 days. On completion of the incubation the proportion of viable cells was counted in a Gorjaev's chamber after staining with a 0.4% trypan blue solution and the accumulation level of virus-specific protein p24 was controlled by the immunoenzyme method as described [Fedyuk N. V., Konovalov E. E., Loktev V. B., Uryvaev L. V., Kulyandin S. A., Pokrovsky A. G.// Problems of Virology, 1992, v. 3, p. 135]. Based on the experimental quantitative data a dose-dependence plot was constructed, from which the inhibiting dose (ID₅₀) -which is the concentration of dipeptide **1** reducing by 50% the accumulation of the virus antigen p24 compared to the control was determined. The inhibiting dose for dipeptide **1** is 0.0012 µM. The therapeutic index or the selectivity index (IS) calculated as a ratio of the cytotoxic dose to the inhibiting dose (CD_{50/}ID₅₀) is 5667 for dipeptide **1.**

In addition, the inhibiting dose ID₉₀that corresponds to the concentration of dipeptide **1** suppressing the virus production by 90% was determined. The inhibiting dose for the compound studied is 0.10 µM. PD₅₀, the concentration of dipeptide **1** protecting by 50% the infected cells from the virus-induced cytopathic effect was also calculated as described earlier by us [Svinarchuk P.P., Konevetz D.A., Pliasunova O.A., Pokrovsky A.G., Vlassov V.V.// Biochimie, 1993,Bd.75,S.243]. PD₅₀ for dipeptide **1** is 0.015 µM. Additionally, the selectivity index (IS) calculated as a ratio of the toxic dose of dipeptide **1** to the protecting dose PD₅₀ (CD₅₀/PD₅₀) thereof was determined, which index is 460. The quantitative data on inhibiting the virus reproduction are listed in Table 3. According to the data of Table 3, dipeptide **1** added simultaneously with the virus, affords 50% inhibition of the virus reproduction at a concentration of 0.0012 µM with a selectivity index (IS) of 5667. Comparing the concentrations inducing 50 and 90% inhibition of virus accumulation provides an important characteristic of the anti-viral activity. From the data of Table 3 it is apparent that the concentration of inventive dipeptide **1** resulting in a 90% virus inhibition is 0.10 µM, which is substantially lower than the corresponding concentration of AZT and dipeptide **9** described earlier. Moreover, the selectivity index (IS) of the inventive dipeptide 1 turned out to be substantially higher compared to that of another known anti-viral agent - **compound 4** which is a structural analog of the compound of the present invention. Depending on an HIV-1 strain used the compound **4** exhibited ID₅₀ in the MT-4 cell culture within the range of 0.045 to 0.75 µM (Y.Kashiwada, Hashimoto F., Cosentino L.M., Chen C.-H., Garrett P.E., Lee K.-H., J.Med.Chem., 1996, V. 39, 1016).

**Table 3. Anti-HIV activity of dipeptide when added simultaneously with the virus in the MT-4 cell culture.**

| Compound | Inhibition of P24 accumulation | | IS |
|---|---|---|---|
| | ID₅₀, µM | ID₉₀, µM | |
| **1** | 0,0012 | 0,1 | 5667 |
| **9** | 0,0026 | 0,198 | 2135 |
| AZT | 0,037 | 1,87 | 1081 |

As is apparent from the above- presented experimental data, an undoubted advantage of the compound of the instant invention is a possibility to use it as a peroral medicinal agent, which results from the low values of inhibiting concentrations towards HIV.

### EXAMPLE 8. Effect of dipeptide 1 on T-cellular immunity under immunization with a strong antigen.

The effect on stimulating T-cellular immunity upon injection with a strong antigen of sheep red blood cells (SRBC) was studied by the rosette formation method. Incomplete Freund's adjuvant (IFA) and glycyrrhizinic acid (GA) which is a known immunostimulant were used as reference preparations.

Outbred male mice weighing 20-22 g were used in the experiment. A suspension of sheep red blood cells (SRBC, 10⁷ cells per mouse) was injected to the animals subcutaneously with 200 µM (10 mg/kg) of the dipeptide **1** under study in 0.5 ml of a physiological saline. SRBC without dipeptide **1** (negative control) or SRBC with IFA or with GA (positive control) were injected to the control animals; another control group - preimmune animals. At day 6 post immunization a suspension of splenocytes was prepared at a concentration of 10 living cells per 1 milliliter. 0.9 ml of the splenocyte suspension and 0.1 ml of a suspension of sheep red blood cells (10⁹ cells per milliliter) were placed in glass vials, mixed and incubated for 16 h at 4°C. After incubation, 4 ml of a cold medium was added into the glass vial, carefully mixed; 100 µl of cell suspension was withdrawn using a pipette with a wide-bore mouth, stained with a 0.4% trypan blue solution and the number of rosettes was counted in a Gorjaev's chamber [Friemel H. Immunological methods/ Translated from German, edited by M. A. Frolova. - Moscow: Mir, 1979, 518 p.]. The results are presented as the number of rosettes formed per 10 cells. The stimulation index was calculated as a ratio of the number of rosettes in the experiment (immunization with SRBC in a mixture with the compound studied) and the number of rosettes in the control (immunization with SRBC alone).

The data are listed in Table 4. As can be seen from Table 4, dipeptide **1** stimulates rosette formation with the stimulation index corresponding to that for GA.

**Table 4. Effect of dipeptide on cellular immune response upon immunization with sheep red blood cells (SRBC)**

| **Compound** | **Number of rosettes per 10³ cells** | **Stimulation index** |
|---|---|---|
| **1** | 24±1,3 | 2,0 |
| **9** | 21±1,4 | 1,8 |
| GA | 24±1,1 | 2,0 |
| SRBC without the preparation | 12±2,9 | 1,0 |
| IFA | 18±2,2 | 1,5 |
| Preimmune control | 2,0±0,8 | 2,0 |

### Example 9. Effect of dipeptide 1 on B-cellular immunity upon immunization with a weak antigen.

The effect of dipeptide 1 on the stimulation of B-cellular response was assessed on the basis of an increase in the titer of specific antibodies after immunization with a weak antigen - bovine serum albumin (BSA). Aluminum hydroxide conventionally used in vaccines as an adjuvant was used as a reference preparation.

400 µg of BSA was injected subcutaneously to the animals in 0.5 ml of 0.9% NaCl. Three groups of control animals were involved. One group was immunized with BSA with IFA (1:1 by volume), the second one - with BSA with 1% Al(OH)₃, the third one - with BSA without the preparation. Additionally, glycyrrhizinic acid (GA) was used as a known immunomodulator. Preimmune mice served as a control. At day 21 the second immunization was performed similarly (the injection solution contained 100 µg of BSA and 200 µg of the test compound). At day 10 post the second immunization blood sera were collected. The titer of antibodies was determined by an enzyme-linked immunoassay technique according to the procedure as described [Enzyme-linked immunoassay technique // Edited by T. T. Igo, G. Lenkhoffaju - Moscow: Mir, 1998]. The quantitative data are listed in Table 5.

**Table 5. Effect of dipeptide 1 on specific humoral immune response after immunization with BSA**

| **Compound** | **Antibody titer (inverse value)** | **Immunogenicity coefficient** |
|---|---|---|
| **1** | 102400 | 512,0 |
| **9** | 51200 | 256 |
| GA | 5120 | 25,6 |
| BSA without the preparation | 200 | 1,0 |
| Al(OH)₃ | 102400 | 512,0 |
| IFA | 102400 | 512,0 |
| Control | 20 | |

As can be seen from Table 5, upon immunization with a weak antigen (BSA) dipeptide **1** exhibited a pronounced immunostimulating activity: the titer of specific antibodies increased 512-fold compared to the immunization without preparations and is comparable with an immunostimulating effect of Al(OH)₃ and IFA.

### Example 10. Determination of acute toxicity of dipeptide 1

The acute toxicity for dipeptide **1** was determined in white outbred mice of both sexes. 0.5 ml of the compound was injected intraperitoneally to mice weighing 20 g, each group comprising 4 animals. 0.5 ml of sterile sunflower oil was injected to the control mice. LD₅₀ of dipeptide **1** of the present invention was more than 2500 mg/kg, which corresponds to class 3 of moderately hazardous substances.

The specific reagents, conditions for conducting reactions, used materials and methods described herein relate to the preferred embodiments, being given simply as examples and are, of course, not intended to limit the scope of this invention to these examples only.

## Claims

1. N'-{N-[3-oxo-lupan-28-oyl]-9-aminononanoyl}-3-amino-3-phenylpropionic acid of formula (1): 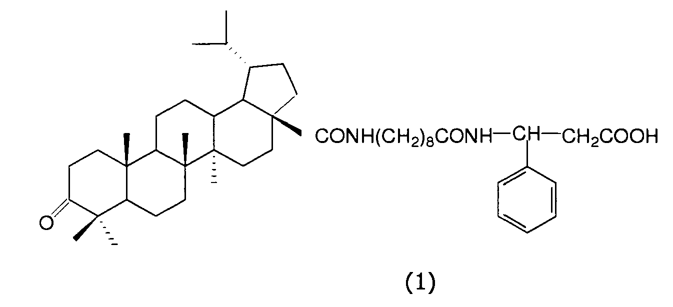 or a pharmaceutically acceptable salt thereof.

2. The compound according to claim 1, wherein the pharmaceutically acceptable salt is selected from the group consisting of a lithium, ammonium, sodium, potassium or calcium salt.

3. A method for producing a compound according to claims 1 or 2, said method comprising the steps of:
a) providing a starting material containing betulin (8);
b) subjecting betulin of formula (8) to catalytic hydrogenation to form dihydrobetulin (**16**);
c) oxidizing dihydrobetulin **(16)** to form dihydrobetulonic acid (10);
d) isolating and purifying the resulting dihydrobetulonic acid (10) through a salt;
e) treating the resulting dihydrobetulonic acid (10) with oxalyl chloride to form acyl chloride (**13**);
f) condensing acyl chloride (**13**) with 9-aminononanoic acid to form amide (12);
g) bringing the resulting amide (12) into reaction with 3-phenyl-3-aminopropionic acid methyl ester to form dipeptide methyl ester (**14**);
h) hydrolyzing dipeptide methyl ester (**14**) to obtain the acid of formula (1);
i) optionally converting the acid of formula (1) into a pharmaceutically acceptable salt; as shown by the following reaction scheme:.

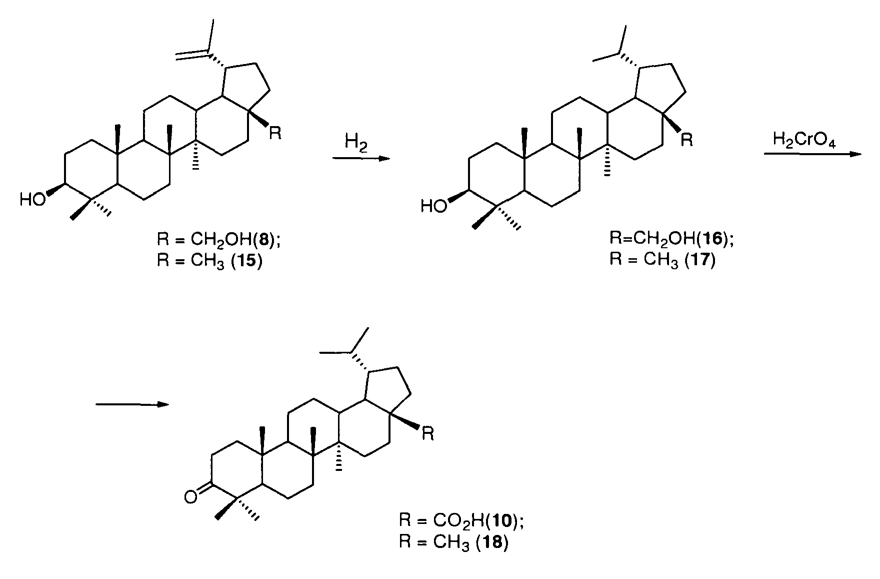

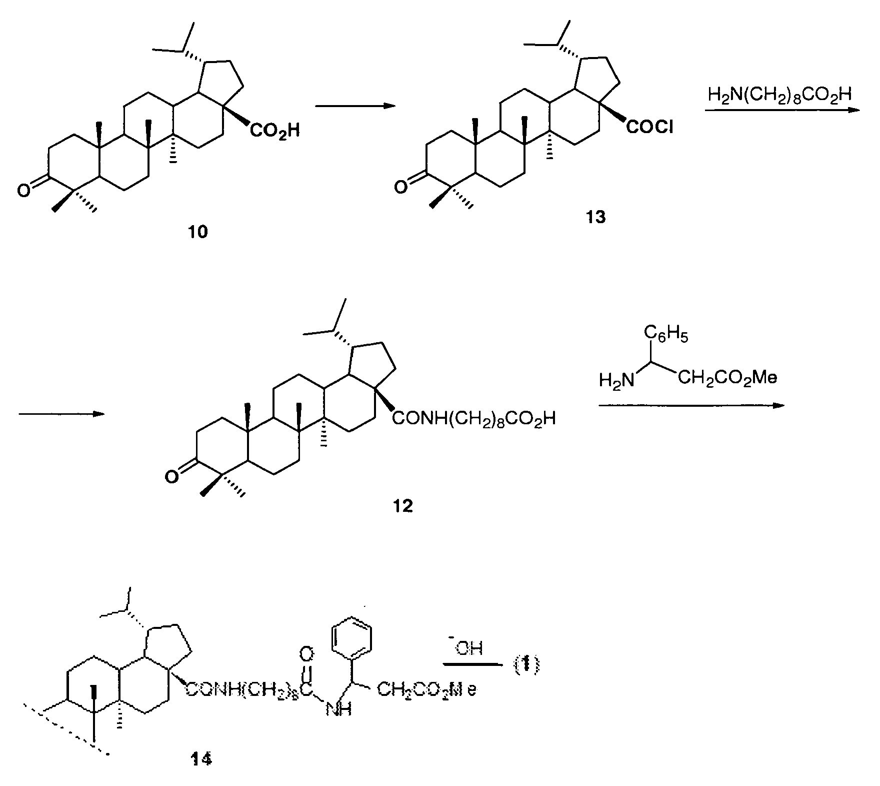

4. The method according to claim 3, comprising using a betulin compound or a total product obtained from birch bark containing 85-90% of betulin and 8-10% of lupeol (**15**) as a starting material.

5. The method according to claims 3 or 4, wherein oxidizing dihydrobetulin (**16**) is carried out with chromic acid.

6. The method according to any one of claims 3 to 5, wherein obtaining a pharmaceutically acceptable salt is carried out by reacting the acid (1) with an equivalent amount of alkaline metal hydroxide or alkaline-earth metal hydroxide or ammonium hydroxide in a solvent chosen from among C1-5 alkyl alcohols or a mixture thereof.

7. A pharmaceutical composition comprising an effective amount of N'-{N-[3-oxo-lupan-28-oyl]-9-aminononanoyl}-3-amino-3-phenylpropionic acid or a pharmaceutically acceptable salt thereof together with a pharmaceutically acceptable carrier, diluent or excipient.

8. N'-{N-[3-oxo-lupan-28-oyl]-9-aminononanoyl}-3-amino-3-phenylpropionic acid or a pharmaceutically acceptable salt thereof for use as an anti-viral agent, preferably as an anti-HIV agent, and as an immunostimulating agent.

## Patentansprüche

1. N'-[N-(3-Oxolupan-28-oyl)-9-aminononanoyl]-3-amino-3-phenylpropionsäure der folgenden Formel (1): 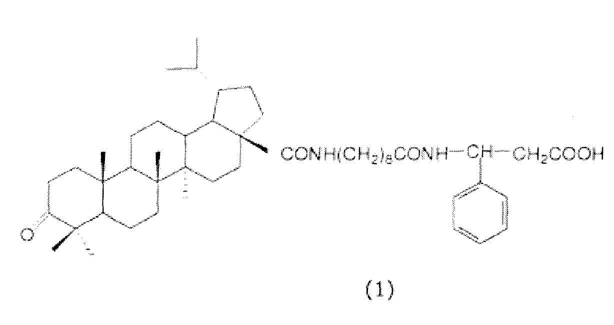 oder ein pharmazeutisch akzeptables Salz derselben.

2. Verbindung nach Anspruch 1, wobei das pharmazeutisch akzeptable Salz aus der Gruppe ausgewählt ist, die aus einem Lithium-, Ammonium-, Natrium-, Kalium- oder Calciumsalz besteht.

3. Verfahren zur Herstellung einer Verbindung nach Anspruch 1 oder 2, wobei das Verfahren die folgenden Schritte umfaßt:
a) Bereitstellen eines Ausgangsmaterials, das Betulin (8) enthält,
b) Vornehmen einer katalytischen Hydrierung an dem Betulin der Formel (8), um Dihydrobetulin (16) zu bilden,
c) Oxidieren des Dihydrobetulins (16), um Dihydrobetulonsäure (10) zu bilden,
d) Isolieren und Reinigen der entstandenen Dihydrobetulonsäure (10) durch ein Salz,
e) Behandeln der entstandenen Dihydrobetulonsäure (10) mit Oxalylchlorid, um ein Acylchlorid (13) zu bilden,
f) Kondensieren des Acylchlorids (13) mit 9-Aminononansäure, um ein Amid (12) zu bilden,
g) Zur-Reaktion-Bringen des entstandenen Amids (12) mit 3-Phenyl-3-aminopropionsäuremethylester, um einen Dipeptidmethylester (14) zu bilden,
h) Hydrolysierten des Dipeptidmethylesters (14), um die Säure der Formel (1) zu erhalten,
i) gegebenenfalls Umwandeln der Säure der Formel (1) in ein pharmazeutisch akzeptables Salz, wie durch das folgende Reaktionsschema dargestellt:

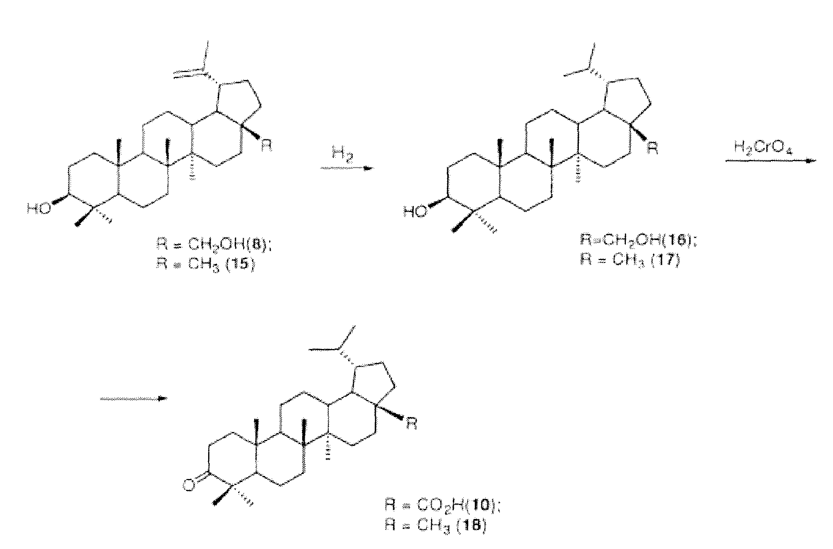

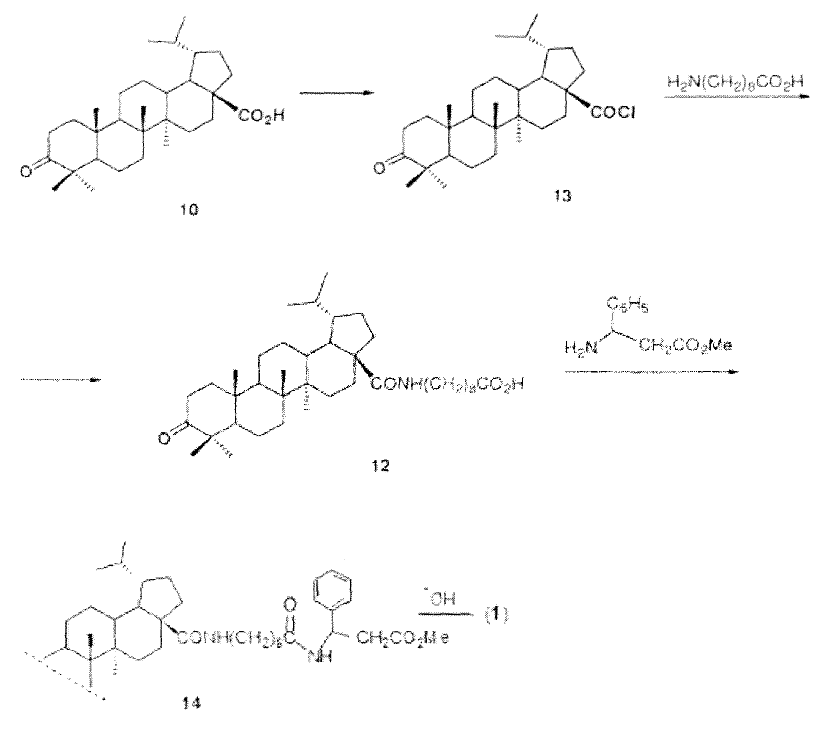

4. Verfahren nach Anspruch 3, welches das Verwenden einer Betulinverbindung oder eines Gesamtprodukts als Ausgangsmaterial umfaßt, das aus Birkenrinde erhalten wird und 85 bis 90 % Betulin und 8 bis 10 % Lupeol (15) enthält.

5. Verfahren nach Anspruch 3 oder 4, wobei das Oxidieren des Dihydrobetulins (16) mit Chromsäure durchgeführt wird.

6. Verfahren nach einem der Ansprüche 3 bis 5, wobei das Erhalten eines pharmazeutisch akzeptablen Salzes durch Umsetzen der Säure (1) mit einer äquivalenten Menge Alkalimetallhydroxid oder Erdalkalimetallhydroxid oder Ammoniumhydroxid in einem Lösungsmittel erfolgt, das aus C₁₋₅-Alkylalkoholen und einem Gemisch derselben ausgewählt wird.

7. Pharmazeutische Zusammensetzung, welche eine wirksame Menge N'-[N-(3-Oxolupan-28-oyl)-9-aminononanoyl]-3-amino-3-phenylpropionsäure oder eines pharmazeutisch akzeptablen Salzes derselben zusammen mit einem pharmazeutisch akzeptablen Träger oder Verdünnungsmittel oder einer pharmazeutisch akzeptablen Salbengrundlage umfaßt.

8. N'-[N-(3-Oxotupan-28-oyl)-9-aminononanoy)]-3-amino-3-phenylpropionsäure oder ein pharmazeutisch akzeptables Salz derselben zur Verwendung als antivirales Mittel, vorzugsweise als Anti-HIV-Mittel, und als Immunstimulans.

## Revendications

1. Acide N'-{N-[3-oxo-lupan-28-oyl]-9-aminononanoyl}-3-amino-3-phénylpropionique de formule (1) : 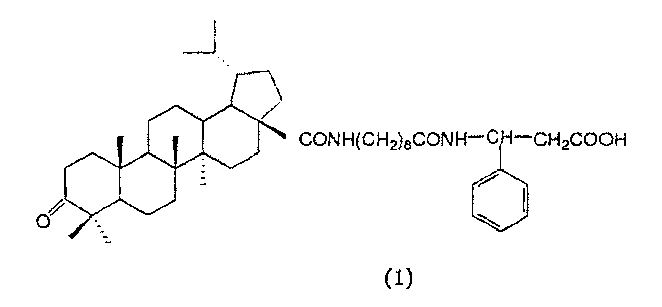 ou sel pharmaceutiquement acceptable de celui-ci.

2. Composé selon la revendication 1 où le sel pharmaceutiquement acceptable est choisi dans le groupe consistant en un sel de lithium, d'ammonium, de sodium, de potassium ou de calcium.

3. Procédé pour produire un composé selon les revendications 1 ou 2, ledit procédé comprenant les étapes de :
a) fournir un produit de départ contenant de la bétuline (8) ;
b) soumettre la bétuline de formule (8) à une hydrogénation catalytique pour former de la dihydrobétuline (16) ;
c) oxyder la dihydrobétuline (16) pour former de l'acide dihydrobétulonique (10) ;
d) isoler et purifier l'acide dihydrobétulonique (10) résultant par le biais d'un sel ;
e) traiter l'acide dihydrobétulonique (10) résultant avec le chlorure d'oxalyle pour former le chlorure d'acyle (13) ;
f) condenser le chlorure d'acyle (13) avec l'acide 9-aminononanoïque pour former l'amide (12) ;
g) mettre l'amide (12) résultant à réagir avec le méthylester d'acide 3-phényl-3-aminopropionique pour former le méthylester dipeptidique (14) ;
h) hydrolyser le méthylester dipeptidique (14) pour obtenir l'acide de formule (1);
i) éventuellement convertir l'acide de formule (1) en un sel pharmaceutiquement acceptable comme le montre le schéma réactionnel suivant :

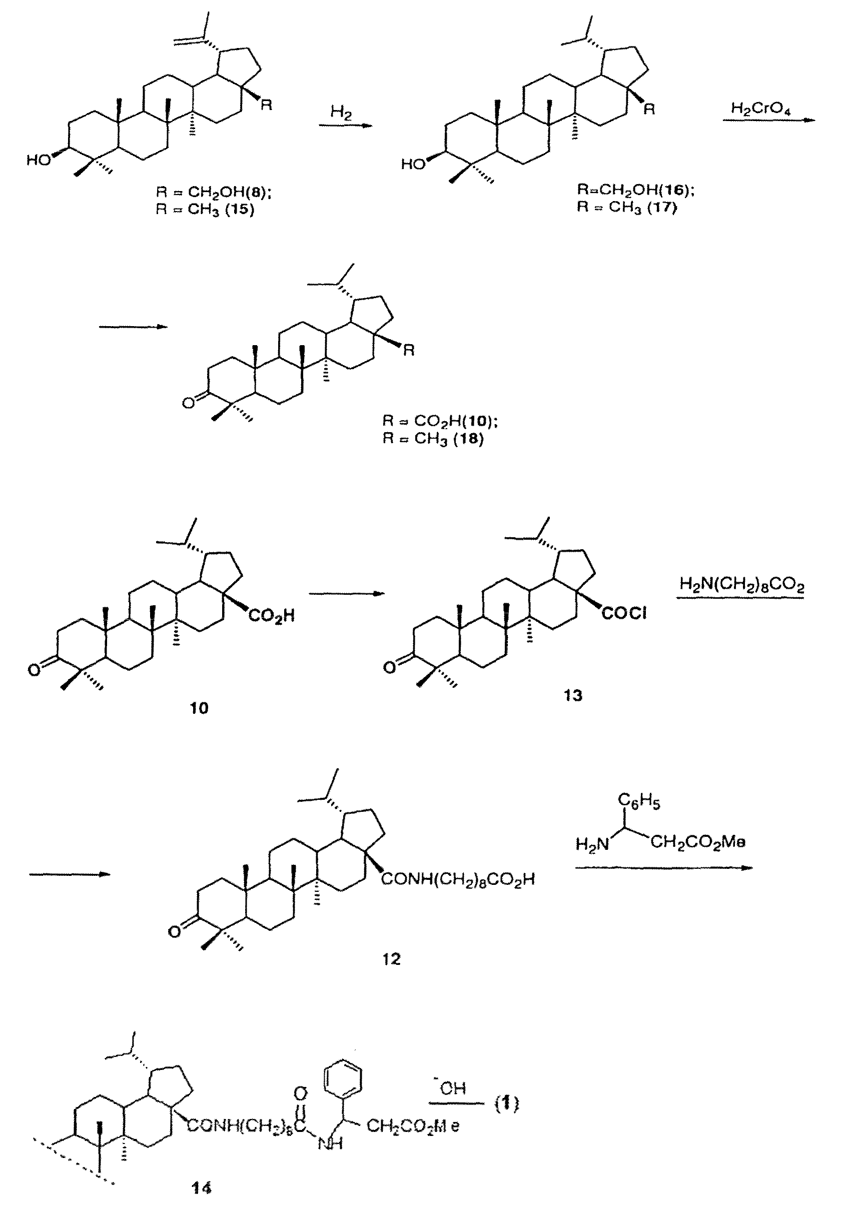

4. Procédé selon la revendication 3 comprenant l'utilisation d'un composé de bétuline ou d'un produit total obtenir à partir de l'écorce de bouleau comprenant 85-90 % de bétuline et 8-10 % de lupéol (15) comme produit de départ.

5. Procédé selon les revendications 3 ou 4 où l'oxydation de la dihydrobétuline (16) est conduite avec l'acide chromique.

6. Procédé selon l'une quelconque des revendications 3 à 5 où l'obtention d'un sel pharmaceutiquement acceptable est réalisée par réaction de l'acide (1) avec une quantité équivalente d'hydroxyde de métal alcalin ou d'hydroxyde de métal alcalino-terreux ou d'hydroxyde d'ammonium dans un solvant choisi parmi les C1-5 alkylalcools ou un mélange de ceux-ci.

7. Composition pharmaceutique comprenant une quantité efficace d'acide N'-{N-[3-oxo-lupan-28-oyl]-9-aminononanoyl}-3-amino-3-phénylpropionique ou d'un sel pharmaceutiquement acceptable de celui-ci ainsi qu'un vecteur, diluant ou excipient pharmaceutiquement acceptable.

8. Acide N'-{N-[3-oxo-lupan-28-oyl]-9-aminononanoyl}-3-amino-3-phénylpropionique ou sel pharmaceutiquement acceptable de celui-ci destiné à être utilisé comme agent antiviral, de préférence comme agent anti-VIH, et comme agent immunostimulant.
